# EUROPEAN PATENT SPECIFICATION

(11) **EP 3 305 112 B1**
(45) Date of publication and mention of the grant of the patent: **25.11.2020**
(21) Application number: 18152200.4
(22) Date of filing: 17.01.2018
(51) Int. Cl.: A24F 47/00

(54) **ATOMIZER**
ZERSTÄUBER
ATOMISEUR

(30) Priority: 19.01.2017 CN 201720077532 U
(43) Date of publication of application: 11.04.2018
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN)
(74) Representative: Proi World Intellectual Property GmbH

(56) References cited:
- WO-A1-2012/129812
- CN-A- 105 901 775
- US-A1- 2013 199 528
- US-A1- 2015 027 456

## Description

### TECHNICAL FIELD

The present disclosure relates to the field of electronic cigarettes, and in particular, to an atomizer.

### BACKGROUND ART

As a substitute of traditional cigarettes, electronic cigarettes are also named virtual cigarettes that are receiving more and more concerns and becoming more and more popular for they have advantages such as safety, no secondhand smoke, no open flames and no fire hazards in use.

In a typical electronic cigarette, the atomizer adopts electrical heating manner for atomization. Known to the inventors, the electronic cigarette conveys tobacco liquid to the heating element for atomization, to generate an aerosol, however which can't control the volume of tobacco liquid conveyed, or convey measurable tobacco liquid.

Document US A1 2013/199528 discloses a medicant delivery system with a heater.

### SUMMARY

In view of the drawbacks in the electronic cigarette known to the inventors, the technical problem to be solved by the present disclosure is providing an atomizer which is capable of controlling the volume of tobacco liquid conveyed or conveying measurable tobacco liquid, in a process that an electronic cigarette conveys tobacco liquid to the heating element for atomization to generate an aerosol, by means of fibers or a porous liquid conducting body.

An atomizer includes an atomization assembly that includes a heating element, configured for heating and atomizing tobacco liquid to generate an aerosol; a liquid storage chamber, configured for storing the tobacco liquid; and a liquid drive component, connected with the tobacco liquid reservoir directly or indirectly, the liquid drive component being configured for controlling a volume of tobacco liquid conveyed to the heating element.

According to the present disclosure, the liquid drive component includes a forced pump and a forced button, when the forced button is pressed, the forced pump conveys the tobacco liquid from the tobacco liquid reservoir to the heating element.

According to the present disclosure, the forced pump includes an inlet and an outlet, the forced pump is in communication with the tobacco liquid reservoir with the inlet extending toward a bottom of the liquid storage chamber; the forced pump conveys the tobacco liquid in the tobacco liquid reservoir from the inlet to the outlet by means of pressing the forced button.

According to embodiments of the present disclosure, the atomizer further includes a U-shaped liquid conducting pipe, one end of the U-shaped liquid conducting pipe is in communication with the outlet of the forced pump, the other end thereof is disposed close to the heating element.

According to embodiments of the present disclosure, the liquid drive component includes an electric pump that is disposed outside the liquid storage chamber, the electric pump conveys the tobacco liquid from the tobacco liquid reservoir to the heating element.

According to embodiments of the present disclosure, the atomizer further includes a liquid conducting pipe, one end of the liquid conducting pipe is in communication with the outlet of the electric pump, the other end thereof is disposed close to the heating element; the electric pump is disposed on the liquid conducting pipe, for conveying the tobacco liquid from the tobacco liquid reservoir to the liquid conducting pipe, further to the heating element.

According to embodiments of the present disclosure, the atomizer further includes a power supply assembly that is coupled with the liquid drive component and the heating element respectively, for driving the liquid drive component while supplying power to the heating element.

According to the present disclosure, the heating element is at least one metal net or one grille metal sheet layered.

According to embodiments of the present disclosure, the heating element further includes a protective layer, encapsulating the heating element, and the protective layer being configured for separating the heating element from the tobacco liquid conveyed from the tobacco liquid reservoir and transmitting heat produced by the heating element.

According to embodiments of the present disclosure, the protective layer is a quartz glass layer, the heating element is capable of generating thermal radiation and infrared radiation though the quartz glass layer, to heat the tobacco liquid.

The present disclosure relates to the atomizer incorporating the liquid drive component, the liquid drive component is capable of controlling the volume of tobacco liquid conveyed to the heating element, and hence conveying measurable tobacco liquid as well as generating measurable aerosol.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a cross-sectional view of an atomizer according to a first embodiment of the present disclosure.
FIG. 2 is an enlarged view of point A in Fig. 1.
FIG. 3 is a cross-sectional view of an atomizer according to a second embodiment of the present disclosure.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

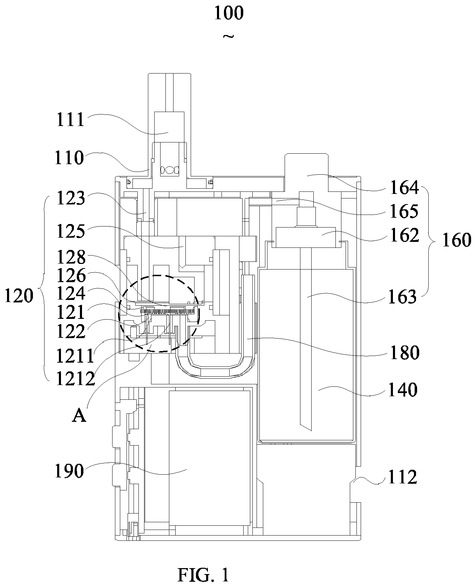

Referring to Fig. 1, Fig. 1 is a cross-sectional view of an atomizer according to a first embodiment of the present disclosure.

As shown in Fig. 1, to understand easily, the cross-sectional view of the atomizer 100 is taken for illustration. The atomizer 100 includes a housing 110, an atomization assembly 120 disposed inside the housing 110, a tobacco liquid reservoir 140 and a liquid drive component 160. Selectively, the atomizer 100 further includes a liquid conducting pipe 180 and a power supply assembly 190.

In which, the atomization assembly 120 includes a heating element 121. The tobacco liquid reservoir 140 is configured for storing tobacco liquid. The liquid drive component 160 is connected with the tobacco liquid reservoir 140 directly or indirectly, configured for controlling the volume of tobacco liquid conveyed from the tobacco liquid reservoir 140 to the heating element 121.

Due to the liquid drive component 160 set on the atomizer 100 of the present disclosure, the liquid drive component 160 is capable of controlling the volume of tobacco liquid conveyed from the tobacco liquid reservoir 140 to the heating element 121, to convey measurable tobacco liquid and hence generate measurable aerosol.

More specifically, the atomization assembly 120 is disposed inside the housing, including a heating element 121, an atomization base 122, a side plate 124, and a cover 126 covering the side plate 124. In which, the side plate 124 is defined to be round, the atomization base 122 and the cover 126 are respectively disposed on two ends of the round side plate 124 to form an atomization cavity128. The cover 126 and/or the side plate 124 have been provided thereon with an aerosol pipe 123 and an air inlet passage 125. As shown in Fig. 1, the aerosol pipe 123 and air inlet passage 125 are set above the cover 126. Particularly, the aerosol pipe 123 is in communication with the mouthpiece 111, air get into the air inlet passage 125 though an air inlet 112 set on the housing 110 and pass though the atomization cavity 128, which may form an airflow passage.

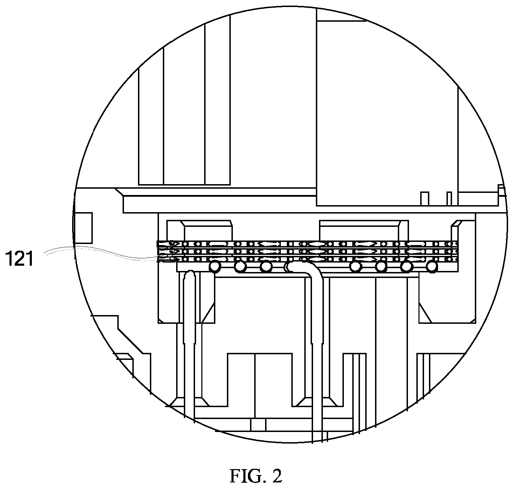

The heating element 121 is disposed in the atomization cavity 128, between or at the side of the air inlet passage 125 and the aerosol pipe 123. As shown in Fig. 1, the heating element 121 is disposed at the side of the air inlet passage 125 and fixedly mounted on the atomization base 122. In some embodiments, the heating element 121 is an exothermic metal or alloy, shaped as a metal wire, a metal net or a grille metal sheet etc.. Selectively, the heating element 121 may be at least one metal net for avoiding tobacco liquid splashing and confining tobacco liquid in the exothermic area, with consequently promoting atomization of tobacco liquid. With reference to Fig. 2, the metal nets that the heating element 121 is may be layered for avoiding tobacco liquid splashing and confining tobacco liquid in the exothermic are, with consequently promoting atomization of tobacco liquid.

In some embodiments, the liquid drive component 160 includes a forced pump 162 and a forced button 164. When the force button 164 is pressed, the forced pump 162 is worked on to convey tobacco liquid from the tobacco liquid reservoir 140 to the heating element 121. The forced button 164 protrudes out beyond the housing 110, the forced pump 162 controls the volume of outputted tobacco liquid based on duration or strength of pressing the forced button 164, the longer or stronger of pressing, the more volume of outputted tobacco liquid while time of the heating element 121 atomizing tobacco liquid is longer. In contrast, the shorter or weaker of pressing, the less volume of outputted tobacco liquid while time of the heating element 121 atomizing tobacco liquid is shorter. In practical experiences, press one time and the atomizer 100 may be sucked for 3-5 seconds.

The forced pump 162 includes an inlet 163 and an outlet 165, the forced pump 162 is in sleeved connection with the tobacco liquid reservoir 140. The forced pump 162 is connected with an upper part of the tobacco liquid reservoir 140 via a thread. The inlet 163 extends toward a bottom of the tobacco liquid reservoir 140. The forced pump 162 conveys tobacco liquid in the tobacco liquid reservoir 140 from the inlet 163 to the outlet 165, further to the heating element 121 after flowing though the liquid conducting pipe 180.

The liquid conducting pipe 180 is a U-shaped liquid conducting pipe, the material thereof is plastic. One end of the U-shaped liquid conducting pipe 180 is in communication with the outlet 165 of the forced pump 162, the other end thereof is disposed close to the heating element 121. In which, the end of the U-shaped liquid conducting pipe 180 communicated with the outlet 165 is higher than the other end of U-shaped liquid conducting pipe 180 disposed close to the heating element 121, for realizing conveying tobacco liquid from up to down, which is beneficial for spread of the tobacco liquid. When the forced button 164 is pressed, the tobacco liquid is ejected by the liquid conducting pipe 180 to the multilayered metal nets from below, enabling the tobacco liquid to be evenly atomized.

The power supply assembly 190 is further coupled with the liquid drive component 160 and the heating element 121 respectively. The power supply assembly 190 is batteries. Particularly, the atomization assembly 120 further includes a first electrode 1211 and a second electrode 1212, the first electrode 1211 and the second electrode 1212 are respectively connected with the anode and cathode of the power supply assembly 190. The heating element 121generats heat upon the first electrode 1211 and the second electrode 1212 are conducted electricity. Furthermore, the power supply assembly 190 is coupled with the forced button 164 of the liquid drive component 160, when the force button 164 is pressed, at the same time the power supply assembly 190 is worked on to supply power to the heating element 121. Therefore, pressing the forced button 164 works on the liquid drive component 160, while it also works on the power supply assembly 190 to supply power to the heating element 121.

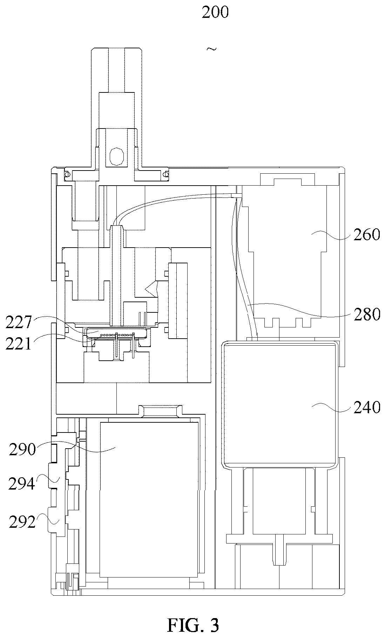

With reference to Fig. 3, Fig. 3 is a cross-sectional view of an atomizer according to a second embodiment of the present disclosure.

As shown in Fig. 3, to understand easily, it takes the cross-sectional view of the atomizer for illustration. The structure of the atomizer in the second embodiment is basically similar with that in the first embodiment, the only difference is that the liquid drive component 260 is an electric pump, the electric pump 260 may be a peristaltic pump disposed outside the tobacco liquid reservoir 240, the electric pump 260 conveys the tobacco liquid in the tobacco liquid reservoir 240 to the heating element 221.

The heating element 221 further includes a protective layer 227 encapsulating the heating element 221, the protective layer 227 is configured for separating the heating element 221 from the tobacco liquid conveyed by the U-shaped liquid conducting pipe 180. There is no chemical reaction between a material of the protective layer 227 and the liquid tobacco, or the protective layer 227 doesn't generate harmful substances after having chemical reaction with the tobacco liquid. In some embodiments, the material of the protective layer 227 includes but not limited to silicon oxide or carbon crystals. Furthermore, in the embodiment, the protective layer 227 is a quartz glass layer capable of transmitting heat produced by the heating element 221 and blocking visible lights but letting infrared lights pass through, as a result, when the heating element 221 generates thermal radiation and infrared radiation though the quartz glass layer, the protective layer 227 heats and atomizes tobacco liquid in the tobacco liquid reservoir 240.

According to embodiments of the present disclosure, the liquid conducting pipe 280 is a U-shaped liquid conducting pipe. One end of the U-shaped liquid conducting pipe 280 is in communication with the tobacco liquid reservoir 240, the other end of the U-shaped liquid conducting pipe 280 is disposed close to the heating element 221. The electric pump 260 is disposed on the U-shaped liquid conducting pipe 280, driving tobacco liquid in the tobacco liquid reservoir 240 to the U-shaped liquid conducting pipe 280, afterwards to the heating element 221.

The power supply assembly 290 is respectively coupled with the liquid drive component 260 and the heating element 221. More specifically, the power supply assembly 290 is coupled with the electric pump 260 and has been provided thereon with a switch button 292 and a control button 294. The switch button 292 is configured for closing and opening the entire atomizer 200. The control button 294 is configured for controlling the working of the electric pump 260. When the control button 294 is pressed, the electric pump 260 is worked on, while the power supply assembly 260 is worked on to supply power to the heating element 221. Therefore, pressing the control button 294 works on the electric pump 260 while it also works on the power supply assembly 260 to supply power to the heating element 221. In practical experiences, continuously press the control button 294 for 3-5 seconds then the atomizer 100 may be sucked.

In some embodiments, the liquid drive component 260 is an electric pump, the electric pump may better control the volume of tobacco liquid, which the controlling process is not related to the present disclosure with consequently no further description herein.

In summary, a person having ordinary skill in the art can understand easily, the atomizer of the present disclosure has been provided thereon with the liquid derive component capable of controlling the volume of tobacco liquid conveyed from the tobacco liquid reservoir to the heating element, and hence conveying measurable tobacco liquid as well as generating measurable aerosol.

## Claims

1. An atomizer (100), comprising:
a heating element (121), configured for heating and atomizing tobacco liquid to generate an aerosol;
a tobacco liquid reservoir (140), configured for storing the tobacco liquid; and a liquid drive component (160), connected with the tobacco liquid reservoir directly or indirectly, the liquid drive component being configured for controlling a volume of tobacco liquid conveyed to the heating element;
wherein the liquid drive component comprises a pump (162) and a button (164), when the button is pressed, the pump conveys the tobacco liquid from the tobacco liquid reservoir to the heating element; wherein the pump comprises an inlet and an outlet; the pump is in communication with the tobacco liquid reservoir; the pump conveys the tobacco liquid in the tobacco liquid reservoir from the inlet to the outlet by means of pressing the button;
**characterized in that**:
the heating element (121) is at least one metal net or one grille metal sheet layered.

2. The atomizer according to claim 1, further comprises a U-shaped liquid conducting pipe (180), one end of the U-shaped liquid conducting pipe is in communication with the outlet of the pump, the other end thereof is disposed close to the heating element.

## Patentansprüche

1. Zerstäuber (100), umfassend:
ein Heizelement (121), das zum Erhitzen und Zerstäuben von Tabakflüssigkeit konfiguriert ist, um Aerosol zu erzeugen;
ein Tabakflüssigkeitsreservoir (140), das zum Speichern der Tabakflüssigkeit konfiguriert ist; und eine Flüssigkeitsantriebskomponente (160), die direkt oder indirekt mit dem Tabakflüssigkeitsreservoir verbunden ist, wobei die Flüssigkeitsantriebskomponente zum Steuern eines dem Heizelement zugeführten Tabakflüssigkeitsvolumens konfiguriert ist;
wobei die Flüssigkeitsantriebskomponente eine Pumpe (162) und einen Knopf (164) umfasst, wobei die Pumpe, wenn der Knopf gedrückt wird, die Tabakflüssigkeit von dem Tabakflüssigkeitsreservoir zu dem Heizelement befördert; wobei die Pumpe einen Einlass und einen Auslass umfasst; die Pumpe mit dem Tabakflüssigkeitsreservoir in Verbindung steht; die Pumpe die Tabakflüssigkeit in dem Tabakflüssigkeitsreservoir mittels Drücken des Knopfes von dem Einlass zu dem Auslass befördert;
**dadurch gekennzeichnet, dass**:
das Heizelement (121) mindestens ein Metallnetz oder ein Gitterblech ist, das geschichtet ist.

2. Zerstäuber nach Anspruch 1 umfasst ferner ein U-förmiges flüssigkeitsleitendes Rohr (180), wobei ein Ende des U-förmigen flüssigkeitsleitenden Rohres mit dem Auslass der Pumpe in Verbindung steht und das andere Ende nahe dem Heizelement angeordnet ist.

## Revendications

1. Un atomiseur (100), comprenant :
un élément chauffant (121), configuré pour chauffer et atomiser le liquide du tabac afin de générer un aérosol ;
un réservoir de liquide de tabac (140), configuré pour stocker le liquide de tabac ; et
un composant d'entra nement liquide (160), connecté au réservoir de liquide de tabac directement ou indirectement, le composant d'entra nement liquide étant configuré pour contrôler un volume de liquide de tabac acheminé vers l'élément chauffant ;
dans lequel le composant d'entra nement de liquide comprend une pompe (162) et un bouton (164), lorsque le bouton est pressé, la pompe transporte le liquide de tabac du réservoir de liquide de tabac à l'élément de chauffage ; dans lequel la pompe comprend une entrée et une sortie ; la pompe est en communication avec le réservoir de liquide de tabac ; la pompe transporte le liquide de tabac dans le réservoir de liquide de tabac de l'entrée à la sortie au moyen d'une pression sur le bouton ;
**caractérisé en ce que** :
l'élément chauffant (121) est au moins un filet métallique ou une grille métallique en couches.

2. L'atomiseur selon la revendication 1, comprend en outre un tube conducteur de liquide en forme de U (180), une extrémité du tube conducteur de liquide en forme de U est en communication avec la sortie de la pompe, l'autre extrémité de celle-ci est disposée à proximité de l'élément chauffant.
